# EUROPÄISCHE PATENTSCHRIFT

(11) **EP 0 363 696 B1**
(45) Veröffentlichungstag und Bekanntmachung des Hinweises auf die Patenterteilung: **26.05.1993**
(21) Anmeldenummer: 89117392.4
(22) Anmeldetag: 20.09.1989
(51) Int. Cl.: A61K 33/00, A61K 33/24, B01F 1/00

(54) **Pharmazeutische Zubereitung sowie Verfahren zu ihrer Herstellung**
Pharmaceutical preparation and process for its manufacture
Préparation pharmaceutique ainsi que procédé pour sa fabrication

(30) Priorität: 22.09.1988 DE 3832211; 05.09.1989 DE 3929411
(43) Veröffentlichungstag der Anmeldung: 18.04.1990
(73) Patentinhaber: Natterer, Siegfried, D-92533 Wernberg-Köblitz (DE)
(72) Erfinder: Natterer, Siegfried, D-92533 Wernberg-Köblitz (DE)
(74) Vertreter: Graf, Helmut, Dipl.-Ing.

(56) Entgegenhaltungen:
- DE-A- 2 517 699
- GB-A- 2 022 998
- US-A- 4 337 245

## Beschreibung

Die Erfindung bezieht sich auf eine pharmazeutische Zubereitung bestehend aus einer Lösung aus Wasser mit metallischen Spurenelementen in Form von Zink und Eisen sowie mit Schwefelsäure als physiologische Säure.

Die Behandlung von Wundzuständen mit anorganischen bzw. mineralischen Wirkstoffen ist prinzipiell bekannt. So wird beispielsweise Zinkoxyd in größerem Umfange sowohl zur Prophylaxe als auch zur Behandlung von Wunden, Verbrennungen usw., also allgemein bei entzündlichen Veränderungen der Haut und des Unterhaut-Zellgewebes, aber auch bei schlecht heilenden Wunden und Ulcera eingesetzt. Trotz der weit verbreiteten Verwendung von Zinkoxyd sind aber die hiermit erzielten Ergebnisse sehr häufig nicht zufriedenstellend.

Bekannt ist speziell auch eine pharmazeutische Zubereitung (GB-A- 20 22 998), die aus einer Lösung aus Wasser und wenigstens einem metallischen Spurenelement oder einem Salz eines solchen Spurenelementes gebildet ist, wobei der pH-Wert der Lösung auf einen Wert kleiner als 4 eingestellt ist.

Der Erfindung liegt die Aufgabe zugrunde, eine pharmazeutische Zubereitung aufzuzeigen, die eine erhöhte Bioverfügbarkeit gewährleistet, und zwar sowohl bei transdermaler als auch bei oraler Anwendung.

Zur Lösung dieser Aufgabe ist eine pharmazeutische Zubereitung erfindungsgemäß so ausgebildet, daß die Lösung einen pH-Wert zwischen 3,0 und 2,0 aufweist und Zink, Eisen und Schwefelsäure jeweils bezogen auf 1 l Lösung in folgenden Anteilen enthält:

| | |
|---|---|
| Zink | 5 - 30 mg |
| Eisen | 10 - 50 mg |
| Schwefelsäure | 100 - 160 mg |

oder

| | |
|---|---|
| Zink in Form von Zinkchlorid | 3 mg |
| Eisen in Form von Eisenchlorid | 6 mg. |

Die erfindungsgemäße Zubereitung, die sowohl für eine orale Anwendung (beispielsweise zur Substitution von Mangel an Spurenelementen) als auch für eine transdermale Anwendung geeignet ist, gewährleistet in jedem Fall eine hohe Bioverfügbarkeit der vorhandenen metallischen Spurenelemente, so daß bereits bei geringen Dosierungen der angestrebte therapeutische Effekt erreicht wird.

Die erfindungsgemäße Zubereitung, die sich auch durch eine gute Schleimhautverträglichkeit auszeichnet, weist insbes. bei transdermaler Anwendung die folgenden pharmakologischen Eigenschaften auf:
entzündungshemmende Wirkung,
antikoagulative Wirkung (insbes. frische Hämatome)
antibakterielle und antivirale Wirkung
Förderung der Wundgranulation bei gleichzeitiger Verminderung von Narbengewebe.

Der Erfindung liegt dabei u.a. die Erkenntnis zugrunde, daß geschädigtes Gewebe einen erhöhten Bedarf an verschiedenen Spurenelementen hat, und dabei insbes. auch an Zink und Eisen. Darüberhinaus besteht aber auch für andere Spurenelemente, insbes. Metalle, in Gewebebereichen mit gesteigerter Aktivität (Abwehrprozesse und/oder Zellprolieferation) ein erhöhter Bedarf. Da nach der der Erfindung zugrundeliegenden Erkenntnis alteriertes Gewebe je nach Grad der Schädigung zunehmend die Fähigkeit verliert, z.B. Zink zu reduzieren, bedeutet dies bei der üblichen Therapie mit Zinkoxydsalben, daß Zink nicht in Lösung geht und deshalb auch nicht oder nur in sehr unzureichendem Umfange in der jeweiligen Wunde zur Wirkung gelangen kann. Im Gegensatz zu derartigen herkömmlichen Zinksalben ist es mit der erfindungsgemäßen Zubereitung aber möglich, Zink, Eisen oder andere essentielle, d.h. körper-notwendige Spurenelemente in saurer Lösung zuzuführen, in der diese Spurenelemente vollständig gelöst sind und somit voll zur Wirkung gelangen können. Gleichzeitig bewirkt der pH-Wert, der zwischen 2 und 3 liegt, ein günstiges biochemisches Milieu für die Aktivität der körpereigenen Abwehr.

Obwohl verdünnte Lösungen von Säuren, insbes. auch von physiologischen Säuren, d.h. dem menschlichen Körper eigene oder hiermit verwandte Säuren, wie beispielsweise Schwefelsäure, Salzsäure, Kieselsäure, Essigsäure und Ascorbinsäure bzw. Zitronensäure im wesentlichen keine pharmakologischen Wirkungen und insbes. auch keine abschwellende Wirkung aufweisen, wenn von einer gewissen antibakteriellen Wirkung einmal abgesehen wird, und obwohl außerdem auch Metalle oder Metallverbindungen nur eine äußerst beschränkte pharmakologische Wirkung besitzen, hat sich gezeigt, daß die die Erfindung auszeichnende Kombination von metallischen Spurenelementen und Säure in wäßriger Lösung zu ganz überraschenden, unerwarteten Wirkungen führt, d.h. aufgrund des Synergieeffektes von Säure und Metall die erfindungsgemäße pharmazeutische Zubereitung, in der die essenziellen metallischen Spurenelemente in Ionen vorliegen, bereits in äußerst geringer Konzentration stark abschwellend, antibakteriell sowie wundheilungsfördernd wirkt. Durch den vorgenannten Synergieeffekt, der auch noch die Verwendung von unterschiedlichen physiologischen Säuren und/oder unterschiedlichen essentiellen, metallischen Spurenelementen gesteigert werden kann, ist es möglich, sehr wirksame pharmazeutische Präparate mit geringer Gesamtkonzentration der Wirkstoffe herzustellen. Die so hergestellten Präparate sind nicht nur hoch wirksam, sondern, wie bereits angesprochen, auch ausgezeichnet verträglich.

In ihrer Darreichungsform kann die erfindungsgemäße Zubereitung dem jeweiligen Indikations- bzw. Anwendungsfall optimal angepaßt werden. So ist es beispielsweise möglich, die erfindungsgemäße Zubereitung als pharmakologisch wirksamer Bestandteil in Salben oder Cremes zu verarbeiten oder aber in flüssiger Darreichungsform auch direkt zur Anwendung zu bringen.

Die metallischen Spurenelemente in der erfindungsgemäßen Zubereitung fördern bei UV-Bestrahlung (natürliche Sonnenbestrahlung oder künstliche UV-Bestrahlung) auch die Bildung von körpereigenem Melanin, was zu einer beschleunigten Bräune der Haut bei UV-Bestrahlung führt. Die erfindungsgemäße Zubereitung eignet sich somit auch als wirksamer Stoff in Bräunungscremes.

Neben der bisher hauptsächlich angesprochenen transdermalen Anwendung zur Behandlung von Wundsein, Verbrennungen sowie anderen entzündlichen Veränderungen der Haut und des Unterhautzellgewebes eignet sich die erfindungsgemäße Zubereitung u.a. auch für eine orale Anwendung insbes. zur Behebung von Mängeln an Spurenelementen bzw. zur Substitution metallischer Spurenelemente im menschlichen oder tierischen Körper, wobei auch in diesem Fall der vorstehend angesprochene Synergieeffekt zwischen Säure und Metall und die hiermit erzielte verbesserte Bioverfügbarkeit zu einer hohen Resorptionsrate führen.

Im praktischen Einsatz hat sich u.a. auch gezeigt, daß bei der oralen Anwendung der erfindungsgemäßen Zubereitung, für die (Anwendung) diese Zubereitung bzw. Lösung hauptsächlich Chloride enthält, die Konzentration an Mineralstoffen bzw. Spurenelementen im Blut (Serumspiegel) vielfach weit stärker ansteigt, als dies aufgrund der Konzentration der Spurenelemente in der Zubereitung bzw. aufgrund der mit ihr verabreichten Menge an Spurenelementen zu erwarten ist. Dieser Effekt ist offenbar darauf zurückzuführen, daß durch die Verabreichung der erfindungsgemäßen Zubereitung, d.h. durch die Verabreichung von Spurenelementen mit Säureüberschuß körpereigene, blockierte Spurenelemente-Depos aktiviert bzw. erschlossen werden. Ein ähnlicher Effekt ist auch bei einer äußeren Anwendung der erfindungsgemäßen Zubereitung festzustellen.

Bei der erfindungsgemäßen Zubereitung eignet sich als Wasser beispielsweise destilliertes Wasser. Bevorzugt wird aber elektrolytisch demineralisiertes Wasser verwendet, da dieses über andere, günstige biologische Eigenschaften verfügt. Die Konzentration der als Salze oder andere wasserlösliche Verbindungen im Wasser gelösten metallischen Spurenelemente sowie das Verhältnis der Spurenelemente zueinander entspricht bevorzugt der physiologischen Menge bzw. Konzentration, d.h. der Konzentration bzw. Menge im natürlichen Serum oder einem Vielfachen hiervon. Der Anteil dieser in größeren Mengen toxischen Metallsalze oder -verbindungen liegt somit im Bereich von Milligramm je Liter Lösung und orientiert sich ebenso wie das Verhältnis der Metallsalze bzw. -verbindungen zueinander in etwa an den physiologischen Mengen. Eine Ausführung der erfindungsgemäßen Zubereitung, mit der sehr gute Ergebnisse erzielt werden, weist Konzentrationen von 3 mg Zink in Form von Zinkchlorid und 6 mg Eisen in Form von Eisenchlorid je Liter Lösung auf.

Eine Erhöhung der Konzentration kann in bestimmten Fällen die Wirksamkeit der Zubereitung erhöhen, wobei sich mit zunehmender Konzentration an metallischen Spurenelementen aber die Verträglichkeit der Zubereitung verschlechtert.

Wie oben bereits erwähnt, ist ein wesentlicher Faktor für die Wirksamkeit der erfindungsgemäßen Zubereitung der pH-Wert. Für die angestrebten pharmakologischen Wirkungen liegt dieser im Bereich zwischen 3,0 und 2,0. Die Einstellung des pH-Wertes erfolgt durch Zugabe einer entsprechenden Menge an physiologischer Säure.

Bei einer anderen Ausführung der Zubereitung gemäß der Erfindung, die (Zubereitung) insbes. zur Behandlung von Entzündungen bei äußerer oder innerer (oraler) Anwendung geeignet ist, enthält in Lösung die metallischen Spurenelemente Zink und Eisen und die Schwefelsäure wiederum in solcher Menge, daß die Lösung einen pH-Wert im Bereich zwischen 2,0 bis 3,0 aufweist. Der Anteil an Zink, Eisen und Schwefelsäure ist dabei so eingestellt, daß 1 l Lösung 5 - 30 mg Zink, 10 - 50 mg Eisen und 100 - 160 mg Schwefelsäure enthält. Bei Anwendung bei Entzündungen erfolgt eine Entgiftung über Sulfate, d.h. die bei Entzündungen vorliegenden bzw. anfallenden giftigen Eiweiß-Verbindungen werden an Sulfate gebunden und damit neutralisiert. Für die äußere Anwendung bildet diese Zubereitung beispielsweise den Wirkstoff einer Salbe. Diese Zubereitung kann aber auch als Wirkstoff für ein zur oralen Einnahme bestimmtes pharmazeutisches Präparat dienen.

Für die Herstellung der erfindungsgemäßen Zubereitung eignen sich grundsätzlich die unterschiedlichsten Verfahren. So kann beispielsweise die Herstellung in der Weise erfolgen, daß die essentiellen, metallischen Spurenelemente Eisen und Zink, gegebenenfalls mit weiteren Metallen der Gruppe Mangan, Chrom, Kupfer, Kobald, Molybdän, Zinn, Vanadium, Nickel, Selen, vorzugsweise in zerkleinerter Form oder in Pulverform mit Säure und Wasser in Lösung gebracht werden, wobei dann der pH-Wert der Lösung beispielsweise durch die zugegebene Menge an Wasser und/oder durch die zugegebene Menge an Säure eingestellt wird.

Weiterhin ist es auch möglich, die erfindungsgemäße Zubereitung dadurch herzustellen, daß wasserlösliche Metallverbindungen der essentiellen metallischen Spurenelemente, nämlich Zinkchlorid und Eisenchlorid, gegebenenfalls zusammen mit Chromoxyd in Wasser unter Zugabe der physiologischen Säure gelöst werden, wobei auch in diesem Fall der erforderliche pH-Wert durch die zugegebene Menge an Wasser und/oder Säure eingestellt wird.

Die erfindungsgemäße Zubereitung kann neben den essentiellen metallischen Spurenelementen auch essentielle nichtmetallische Spurenelemente beispielsweise der Gruppe Silizium, Jod und Fluor enthalten, die in der Zubereitung ebenfalls in einer physiologischen Menge oder einem Vielfachen hiervon enthalten sind.

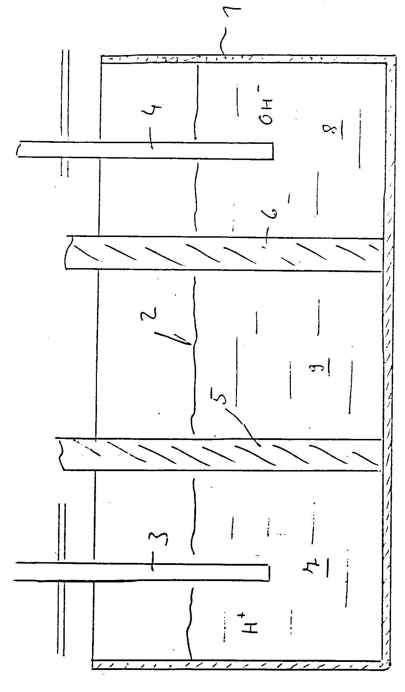

Ein bevorzugtes Verfahren zur Herstellung der erfindungsgemäßen Zubereitung wird nachfolgend anhand der Figur, die in vereinfachter Schnittdarstellung eine Anordnung zum Herstellen der Zubereitung durch Elektrolyse zeigt, näher erläutert.

In dieser Figur ist 1 ein Behälter aus elektrisch nichtleitendem Material, z.B. Glas oder Kunststoff. In diesem Behälter 1 wird eine im wesentlichen neutrale, wässrige Ausgangslösung eingebracht, die in Wasser gelöste Chloride und/oder Sulfate enthält. In diese Ausgangslösung 2 reichen zwei Elektroden 3 und 4 derart hinein, daß diese Elektroden 3 und 4 im Inneren des Behälters mit vorgegebenem Abstand voneinander angeordnet sind. Die beiden Elektroden 3 und 4 sind mit einer nicht näher dargestellten Gleichspannungsquelle so verbunden, daß die Elektrode 3 als Kathode und die Elektrode 4 als Anode wirken. Zwischen den beiden Elektroden sind zwei Membranen bzw. Diaphragmen 5 und 6 angeordnet, so daß durch die diese Membranen 5 und 6 der Innenraum des Behälters 1 im wesentlichen in drei Zonen unterteilt wird, nämlich in die im Bereich der Elektrode 3 bzw. Kathode liegende Zone 7, in die im Bereich der Elektrode 4 bzw. der Anode liegende Zone 8 sowie in die zwischen den Zonen 8 und 7 liegende Zone 9. Die Membranen 5 und 6 sind aus einem geeigneten Material, z.B. aus Ton, Gewebe, filz- oder vliesartigem Material hergestellt und weisen eine solche Dichte auf, daß eine Ionisierung der Ausgangslösung 2 durch die mit der Gleichspannungswelle verbundenen Elektroden 3 und 4 möglich ist, die Membranen 5 und 6 gleichzeitig jedoch eine Konzentration des bei der Ionisierung bzw. Elektrolyse erhaltenen sauren Anteils (H⁺) im Bereich der Zone 7 und eine Konzentrierung des bei der Elektrolyse erhaltenen basischen Anteils (OH⁻) im Bereich der Zone 8 gewährleisten und die Trennung der beiden Zonen 7 und 8 durch die mittlere Zone 9 eine eventuelle Ausfällung verhindert. Mit der dargestellten Anordnung, die eine reproduzierbare Arbeitsweise ermöglicht, besteht die die Kathode bildende Elektrode 3 wenigstens aus den zur Bildung der essenziellen metallischen Spurenelemente geeigneten Metallen bzw. aus einer Legierung solcher Metalle.

Bei der Elektrolyse werden diese, die Elektrode 3 bildenden Metalle in der Zone 7 gelöst, so daß dort eine saure Lösung mit zunehmender Konzentration an den essenziellen, metallischen Spurenelementen erhalten wird.

Selbstverständlich ist es möglich, in der Zone 7 auch mehrere Elektroden 3 aus unterschiedlichen Metallen und/oder aus unterschiedlichen oder gleichen Legierungen vorzusehen. Ebenso ist es möglich, in der Zone 8 mehr als eine Elektrode 4 zu verwenden. Weiterhin ist es auch möglich, zusätzlich zu der Elektrode 3 in der Zone 7 eine weitere, neutrale Elektrode vorzusehen, die im Gegensatz zu der Elektrode 3 oder mehreren derartigen Elektroden 3 nicht in Lösung geht.

Um die angestrebte Konzentration an den metallischen Spurenelementen in der Zone 7 einstellen zu können, sind zumindest die dortige Elektrode oder Elektroden 3 in einer verstellbaren Halterung gehalten, die je nach Bedarf eine Verkleinerung oder Vergrößerung der Elektroden-Eintauchtiefe ermöglicht.

Anstelle der Membranen 5 und 6 kann auch wenigstens eine als Rohr ausgebildete Membrane, vorzugsweise eine solche Membrane Verwendung finden, die aus wasserdurchlässigem, gebranntem Ton gefertigt ist. Die verwendeten Elektroden sind dann innerhalb dieser wenigstens einen Membrane derart angeordnet, daß je nach Anordnung entweder der durch die Elektrolyse saure oder alkalische Anteil im Inneren der jeweils rohrförmigen Membrane konzentriert wird.

## Patentansprüche

1. Pharmazeutische Zubereitung bestehend aus einer Lösung aus Wasser mit metallischen Spurenelementen in Form von Zink und Eisen sowie mit Schwefelsäure als physiologische Säure, **dadurch gekennzeichnet,** daß die Lösung einen pH-Wert zwischen 3,0 und 2,0 aufweist und Zink, Eisen und Schwefelsäure in folgenden Anteilen, jeweils bezogen auf 1 l Lösung, enthält:
| | |
|---|---|
| Zink | 5 - 30 mg |
| Eisen | 10 - 50 mg |
| Schwefelsäure | 100 - 160 mg. |

2. Pharmazeutische Zubereitung bestehend aus einer Lösung aus Wasser mit metallischen Spurenelementen in Form von Zink und Eisen sowie mit Schwefelsäure als physiologische Säure, dadurch gekennzeichnet, daß die Lösung einen pH-Wert zwischen 3,0 und 2,0 aufweist und Zink und Eisen in folgenden Anteilen, jeweils bezogen auf 1 l Lösung enthält:
| | |
|---|---|
| Zink in Form von Zinkchlorid | 3 mg |
| Eisen in Form von Eisenchlorid | 6 mg. |

## Claims

1. Pharmaceutical preparation comprising a solution of water with metallic trace elements in the form of Zinc and Iron as well as Sulphuric acid as physiological acidity characterised in that the solution has a pH-value between 3.0 and 2.0 and contains Zinc, Iron and Sulphuric acid in the following proportions, each time referring to a 1 l solution.
| | |
|---|---|
| Zinc | 5 - 30 mg |
| Iron | 10 - 50 mg |
| Sulphuric acid | 100 - 160 mg |

2. Pharmaceutical preparation comprising a solution of water with metallic trace elements in the form of Zinc and Iron as well as Sulphuric acid as physiological acidity, characterised in that the solution has a pH-value between 3.0 and 2.0 and contains Zinc and Iron in the following proportions, each time referring to a 1 litre solution:
| | |
|---|---|
| Zinc in the form of Zinc Chloride | 3 mg |
| Iron in the form of Iron Chloride | 6 mg |

## Revendications

1. Préparation pharmaceutique constituée d'une solution d'eau avec des oligo-éléments métalliques sous forme de zinc et de fer ainsi qu'avec de l'acide sulfurique comme acide physiologique, caractérisée en ce que la solution a un pH compris entre 3,0 et 2,0 et contient du zinc, du fer et de l'acide sulfurique dans les proportions suivantes chaque fois pour 1 l de solution:
| | |
|---|---|
| zinc | 5 à 30 mg |
| fer | 10 à 50 mg |
| acide sulfurique | 100 à 160 mg. |

2. Préparation pharmaceutique constituée d'une solution d'eau avec des oligo-éléments métalliques sous forme de zinc et de fer ainsi qu'avec de l'acide sulfurique comme acide physiologique, caractérisée en ce que la solution a un pH compris entre 3,0 et 2,0 et contient du zinc et du fer dans les proportions suivantes chaque fois pour 1 l de solution:
| | |
|---|---|
| zinc sous forme de chlorure de zinc | 3 mg |
| fer sous forme de chlorure de fer | 6 mg |
